# FASCICULE DE BREVET EUROPEEN

(11) **EP 1 976 831 B1**
(45) Date de publication et mention de la délivrance du brevet: **27.10.2010**
(21) Numéro de dépôt: 07712654.8
(22) Date de dépôt: 03.01.2007
(51) Int. Cl.: C09D 4/00, C09J 4/00, C09J 133/04, C09D 133/04

(54) **COMPOSITIONS A BASE DE (METH)ACRYLATES D'ALKYLIMIDAZOLIDONE**
ZUSAMMENSETZUNGEN AUF DER BASIS VON ALKYLIMIDAZOLIDON(METH)ACRYLATEN
COMPOSITIONS BASED ON ALKYLIMIDAZOLIDONE (METH)ACRYLATES

(30) Priorité: 23.01.2006 FR 0600570
(43) Date de publication de la demande: 08.10.2008
(73) Titulaire: ARKEMA FRANCE, 92700 Colombes (FR)
(72) Inventeur: PAUL, Jean-Michel, F-57070 Metz (FR)
(74) Mandataire: Bonnel, Claudine
(86) Numéro de dépôt international: PCT/FR2007/050603
(87) Numéro de publication internationale: WO 2007/083045

(56) Documents cités:
- EP-A- 0 902 017
- EP-A- 1 241 163
- EP-A- 1 293 502
- US-A1- 2003 235 686
- FLICK, ERNEST W.: "Plastic Additives 2nd edition" 1993, NOYES PUBLICATIONS , XP002397080 page 431; composé 506

## Description

### Domaine de l'invention

L'invention concerne les monomères (méth)acryliques spéciaux et a trait plus particulièrement à des solutions de (méth)acrylates d'alkylimidazolidone dans des esters (méth)acryliques, ainsi qu'à leur préparation et leurs applications.

### Art antérieur et problème technique

Il est connu, par exemple d'après les documents EP 1 293 502, EP 433 135, EP 712 846, EP 650 962 ou EP 619 309, de préparer les acrylates ou les méthacrylates (désignés par (méth)acrylates) d'alkylimidazolidone de formule (I) : 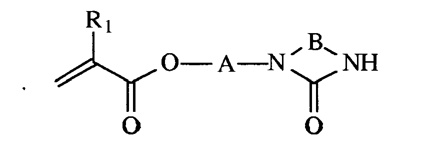 dans laquelle R₁ est un atome d'hydrogène ou un groupement méthyle, et A et B représentent, indépendamment l'un de l'autre, un groupement alkylène à chaîne droite ou ramifiée ayant de 2 à 5 atomes de carbone,
selon un procédé de transestérification par réaction d'au moins un (méth)acrylate d'alkyle de formule (II) : 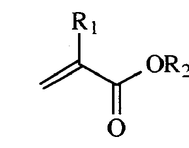 dans laquelle R₁ a la signification précitée et R₂ est un groupement alkyle à chaîne droite ou ramifiée ayant de 1 à 4 atomes de carbone,
avec un alcool hétérocyclique de formule (III) : 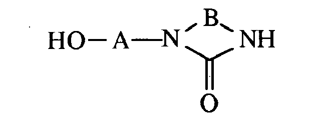 dans laquelle A et B ont les significations précitées,
en présence d'un catalyseur.

Lorsque le (méth)acrylate (II) est en large excès par rapport à l'alcool hétérocyclique (III), on obtient à la fin de la réaction une composition comprenant une solution de (méth)acrylate d'alkylimidazolidone de formule (I) dans le (méth)acrylate (II).

Ainsi, les (méth)acrylates d'alkylimidazolidone sont généralement commercialisés dans un (méth)acrylate léger (II). Plus particulièrement, le méthacrylate d'éthyl-1-imidazolyl-2-one (MEIO), préparé à partir de méthacrylate de méthyle (MAM), est commercialisé en solution dans le méthacrylate de méthyle (MAM).

Il est connu de substituer le méthacrylate de méthyle par de l'eau dans des compositions comprenant un (méth)acrylate d'alkylimidazolidone et du méthacrylate de méthyle, obtenues selon un procédé de transestérification.

Selon le document EP 1 241 163, la méthode consiste à éliminer la quasi-totalité du méthacrylate de méthyle par distillation sous vide dans des conditions de température/pression de 40°C à 60°C / 760 à 60 mm de Hg, puis à ajouter l'eau. La composition finale obtenue contient plus de 50 % d'eau.

Selon le document EP 902 017, la méthode consiste à introduire une petite quantité d'eau dans le mélange comprenant le (méth)acrylate d'alkylimidazolidone et le méthacrylate de méthyle, puis à éliminer le méthacrylate de méthyle par distillation sous forme d'azéotrope avec l'eau, tout en introduisant en continu de l'eau. La composition finale obtenue contient environ 48 % d'eau.

Les (méth)acrylates d'alkylimidazolidone sont connus pour leur rôle dans la constitution de polymères utilisables comme revêtements et adhésifs, dans le domaine du papier et des textiles, pour leur utilisation comme agents de traitements du cuir et dans la production de peintures en émulsion.

Les (méth)acrylates d'alkylimidazolidone en solution dans l'eau ou dans un (méth)acrylate léger, tel que le méthacrylate de méthyle, présentent cependant des inconvénients lors de leur mise en oeuvre. En particulier, selon l'utilisation finale, l'eau présente l'inconvénient d'une évaporation lente et le méthacrylate de méthyle possède une faible tension de vapeur et conduit à l'émission de composés organiques volatiles (VOC).

Il a maintenant été trouvé de nombreux avantages à substituer l'eau ou le (méth)acrylate léger par un monomère (méth)acrylique plus lourd et polymérisant facilement sous radiation, dans les formulations de (méth)acrylates d'alkylimidazolidone dans l'eau ou dans un (méth)acrylate léger. Le monomère (méth)acrylique plus lourd jouant le rôle de solvant réactif et polymérisable sous radiation ou sous faisceau d'électrons, permet de conférer des caractéristiques particulières à la composition ainsi obtenue, notamment d'étendre le domaine d'utilisation des (méth)acrylates d'alkylimidazolidone à celui des formulations polymérisables sous UV ou sous faisceau d'électrons, plus particulièrement dans le domaine des peintures. Les problèmes résultants de l'émission de VOC sont ainsi évités.

### Résumé de l'invention

La présente invention a donc pour objet une composition comprenant un (méth)acrylate d'alkylimidazolidone de formule (I) 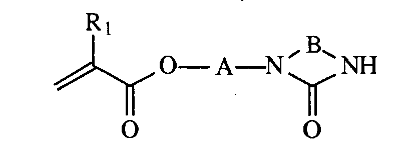 dans laquelle R₁ est un atome d'hydrogène ou un groupement méthyle, et A et B représentent, indépendamment l'un de l'autre, un groupement alkylène à chaîne droite ou ramifiée ayant de 2 à 5 atomes de carbone,
en solution dans un (méth)aérylate de formule (IV) 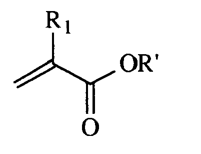 dans laquelle R₁ est un atome d'hydrogène ou un groupement méthyle, et R' est un radical alkyle à chaîne droite ou ramifiée ayant de 9 à 40 atomes de carbone ou un radical aliphatique cyclique, alcényle, aryle, alkyle-aryle ou aryle alkyle ayant de 2 à 40 atomes de carbone, ces radicaux pouvant être substitués et pouvant contenir des hétéroatomes, la composition comprenant en outre un inhibiteur de polymérisation.

Avantageusement, la composition contient de 20 à 80 % en poids de (méth)acrylate d'alkylimidazolidone de formule (I) et de sous-produits monomériques porteurs d'une fonction uréido, de préférence, de 30 à 60 % en poids, et plus particulièrement de 45 à 55 % en poids. Les sous-produits monomériques porteurs d'une fonction uréido sont les sous-produits inhérents à la fabrication du (méth)acrylate d'alkylimidazolidone de formule (I) par transestérification. Ils résultent de l'addition de type Michaël de la fonction amine secondaire du cycle imidazolidone sur une autre molécule de (méth)acrylate d'alkylimidazolidone (I) ou sur une molécule de (méth)acrylate d'alkyle (II). Généralement, ces sous-produits sont présents à une teneur allant de 5 à 25 % par rapport au (méth)acrylate d'alkylimidazolidone, en particulier de 10 à 20 %.

L'invention concerne aussi un procédé de préparation de solutions de (méth)acrylate d'alkylimidazolidone de formule (I) 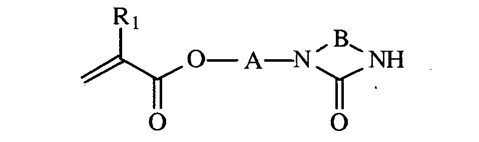 dans laquelle R₁ est un atome d'hydrogène ou un groupement méthyle, et A et B représentent, indépendamment l'un de l'autre, un groupement alkylène à chaîne droite ou ramifiée ayant de 2 à 5 atomes de carbone,
dans un (méth)acrylate de formule (IV) 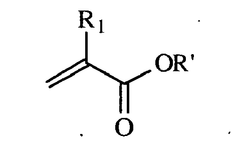 dans laquelle R₁ est un atome d'hydrogène ou un groupement méthyle, et R' est un radical alkyle à chaîne droite ou ramifiée ayant de 9 à 40 atomes de carbone ou un radical aliphatique cyclique, alcényle, aryle, alkyle-aryle ou aryle alkyle ayant de 2 à 40 atomes de carbone, ces radicaux pouvant être substitués et pouvant contenir des hétéroatomes,
à partir de solutions de (méth)acrylate d'alkylimidazolidone de formule (I) dans l'eau ou dans un (méth)acrylate de formule (II) 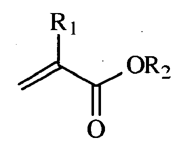 dans laquelle R₁ est un atome d'hydrogène ou un groupement méthyle et R₂ est un groupement alkyle à chaîne droite ou ramifiée ayant de 1 à 4 atomes de carbone.

L'invention a aussi pour objet l'utilisation des compositions précitées dans la préparation de polymères utilisables comme revêtements et d'adhésifs, dans le traitement du papier et des textiles, comme agents de traitement du cuir et dans la production de peintures ou vernis polymérisables par chauffage, ou sous radiation UV ou visible, ou sous faisceau d'électrons.

D'autres caractéristiques et avantages de l'invention ressortiront mieux à la lecture de la description qui suit.

### Exposé détaillé de l'invention

Comme exemples de composé (I), on peut citer ceux pour lesquels le groupement A est un groupement alkylène ayant 2 atomes de carbone, et plus particulièrement le méthacrylate d'éthyl-1- imidazolidyl-2-one (MEIO).

Comme exemples de radicaux R' dans la formule (IV), on peut citer les radicaux lauryle, stéaryle, dodécyle, béhényle, isobornyle, phényle, naphtyle, naphtyloxyalkyle comprenant un groupement alkyle linéaire ou ramifié ayant de 1 à 10 atomes de carbone.

Les acrylates de formule (IV) sont préférés, et plus particulièrement l'acrylate d'isobornyle.

Une composition préférée comprend de 45 à 55 % en poids de MEIO et de sous-produits monomériques porteurs d'une fonction uréido, et de 55 à 45 % de (méth)acrylate d'isobornyle ; de préférence la teneur en MEIO est supérieure à 35 % .

Les compositions selon l'invention peuvent contenir un ou plusieurs inhibiteurs de polymérisation, tel que la phénothiazine, l'hydroquinone l'éther monométhylique d'hydroquinone, le diterbutyl para-crésol, la paraphénylène diamine, le TEMPO (2,2,6,6-tétraméthyl-1-piperidinyloxy) et le di-tertiobutylcatéchol, ou les dérivés du TEMPO. La teneur en inhibiteurs de polymérisation est comprise entre 100 et 2000 ppm par rapport au mélange final, de préférence de 200 à 600 ppm.

Les solutions de (méth)acrylate d'alkylimidazolidone de formule (I) dans un (méth)acrylate (IV) sont préparées à partir de solutions initiales de (méth)acrylate d'alkylimidazolidone de formule (I) dans l'eau ou dans un (méth)acrylate de formule (II) 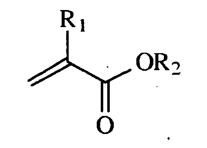 dans laquelle R₁ est un atome d'hydrogène ou un groupement méthyle et R₂ est un groupement alkyle à chaîne droite ou ramifiée ayant de 1 à 4 atomes de carbone.

Le procédé consiste à substituer l'eau ou le (méth)acrylate de formule (II) par un (méth)acrylate de formule (IV).

De préférence, le (méth)acrylate (II) est un (méth)acrylate léger tel que le (méth)acrylate de méthyle ou le (méth)acrylate d'éthyle.

La teneur en (méth)acrylate d'alkylimidazolidone et de sous-produits monomériques porteurs d'une fonction uréido dans la solution initiale n'est pas critique, elle est généralement de 20 à 80 %, de préférence de 40 à 60 % et plus particulièrement de 45 à 55 %. La solution initiale peut être stabilisée avec un ou plusieurs inhibiteurs de polymérisation parmi lesquels on peut citer la phénothiazine, l'hydroquinone l'éther monométhylique d'hydroquinone, le diterbutyl para-crésol, la paraphénylène diamine, le TEMPO (2,2,6,6-tétraméthyl-1-piperidinyloxy) et le di-tertiobutylcatéchol, ou les dérivés du TEMPO.

Les solutions initiales de (méth)acrylate d'alkylimidazolidone de formule (I) dans l'eau ou dans un (méth)acrylate léger de formule (II) peuvent être obtenues selon l'un des procédés connus de l'homme du métier, tels que cités précédemment, ou sont disponibles commercialement.

Le procédé selon l'invention peut être mis en oeuvre selon différents modes de réalisation.

Selon un premier mode de réalisation, la solution de (méth)acrylate d'alkylimidazolidone dans un (méth)acrylate (II), et le (méth)acrylate (IV) de substitution sont chargés initialement dans un réacteur et le (méth)acrylate léger est éliminé par distillation sous vide. La pression est ajustée de façon à ne pas dépasser une température comprise entre 50°C et 80°C dans le mélange lors de la distillation. On peu opérer dans un réacteur agité ou à l'aide d'un évaporateur rotatif ou à film mince.

Selon un second mode de réalisation, la solution de (méth)acrylate d'alkylimidazolidone dans un (méth)acrylate (II) est introduite dans un réacteur et une partie du (méth)acrylate (II) est distillée ; avantageusement, 20 à 70 % du (méth)acrylate (II) présent dans la solution de (méth)acrylate d'alkylimidazolidone, et de préférence 40 à 60 % sont distillés au cours de cette étape. Puis, le (méth)acrylate (IV) de substitution est ajouté en continu, tout en continuant la distillation du (méth)acrylate (II).

On peut procéder de la même façon avec des solutions de (méth)acrylate d'alkylimidazolidone dans l'eau.

Dans les deux modes de réalisation, un bullage d'air, éventuellement appauvri (8 à 9 % O₂) est effectué.

Un strippage final, consistant en un épuisement des composés légers avec de l'air appauvri contenant 8 à 9 % d'O₂ peut être mis en oeuvre permettant de compléter l'élimination du (méth)acrylate (II) dans le mélange.

La teneur résiduelle en (méth)acrylate (II) dans la solution obtenue est généralement inférieure à 5 %, de préférence inférieure à 1 %.

Les compositions selon l'invention et les solutions obtenues selon le procédé de l'invention présentent de nombreux avantages du point de vue de leurs propriétés applicatives, plus particulièrement dans le domaine des peintures, en raison de la présence du (méth)acrylate (IV) qui joue un rôle de solvant réactif, polymérisable par chauffage en présence d'un initiateur radicalaire, ou sous radiation UV ou visible en présence de photoamorceurs, ou sous faisceau d'électrons.

Les exemples suivants illustrent la présente invention sans toutefois en limiter la portée.

### Exemples de réalisation

Les abréviations suivantes y sont utilisées :
HEIO : 1-(2-hydroxyéthyl)-imidazolidyl-2-one
MEIO : méthacrylate d'éthyl-1- imidazolidyl-2-one
NORSOCRYL^{©} 104 : solution à 50 % massique de méthacrylate d'éthyl-1-imidazolidyl-2-one et d'impuretés monomériques du type uréido dans du méthacrylate de méthyle.
MAM : méthacrylate de méthyle
EMHQ : éther méthylique d'hydroquinone
PTZ : phénothiazine
AISOBOR : acrylate d'isobornyle
MAISOBOR : méthacrylate d'isobornyle

Les pourcentages sont exprimés en pourcentages massiques.

### Exemple 1

Dans un appareillage constitué d'un évaporateur rotatif, on charge 1000 g de NORSOCRYL^{©}104 et 480 g d'AISOBOR.

On distille le MAM à une température du mélange de pied de 50°C en ajustant la pression de service (de 60 mm Hg à 15 mmHg en final).

En fin de distillation du MAM, et après refroidissement, on récupère une solution homogène limpide qui contient 42 % de MEIO, 3 % de MAM, 49 % d'AISOBOR, 2 % de HEIO et 4 % d'impuretés.

### Exemple 2

On utilise un évaporateur rotatif de plus grande capacité de type QUICKFIT M200 de capacité 20 litres et de vitesse de rotation 12-95 tours par minute. On charge 8,0 kg de NORSOCRYL^{©}104 qui contient 39,6 % de MEIO, 1,0 % de HEIO, 52,3 % de MAM, 0,017 % de EMHQ et 0,046 % de PTZ.

La température au sein de l'évaporateur est maintenue entre 40 et 56°C en ajustant la pression de service entre 23 et 34 mbar (P absolue). On distille 4,02 kg de MAM en 3h45min.

On charge alors 4,0 kg d'AISOBOR en 15 min. Après 20 min d'agitation, on refroidit le mélange à 30°C, et on le vidange après avoir ramené la pression à la pression atmosphérique. Pendant tout l'essai on maintient un bullage d'air dans le mélange.

On obtient à la fin 7,83 kg de mélange ayant la composition massique suivante:
- MEIO : 37,5 %
- AISOBOR : 54,1%
- MAM : 1,0 %
- Autres : 7,2 %
- EMHQ : 0,023 %
- PTZ : 0,043 %

### Exemple 3

L'exemple 1 est repris en remplaçant l'AISOBOR par du MAISOBOR.

On obtient un mélange contenant :
- MEIO : 38,0 %
- MAISOBOR : 54,0 %
- MAM: 0.5 %
- Autres : 7,5 %
- EMHQ : 0,022 %
- PTZ: 0,044 %

### Exemple 4

La préparation du mélange est ici réalisée dans un réacteur agité chauffé par circulation d'huile dans une double enveloppe surmonté d'une colonne à distiller.

On charge 387 g de NORSOCRYL^{©}104 qui contient 39,6 % de MEIO, 1,0 % de HEIO, 52,3 % de MAM, 0,017 % de EMHQ et 0,046 % de PTZ. On maintient un bullage d'air dans le réacteur durant toute l'opération et on chauffe le milieu à une température qui devra rester comprise entre 70 et 75°C. Pour ce faire, on baisse progressivement la pression de service de 300 mm Hg à 50 mm Hg.

Le MAM est éliminé par distillation. Lorsqu'on a distillé 184 ml de MAM, on commence l'introduction en continu de 186 g d'AISOBOR pendant une heure. Lorsque tout l'AISOBOR a été introduit, on augmente le bullage d'air afin d'éliminer les traces de MAM résiduel. Le strippage est effectué sous 15 mm Hg de pression. Le produit final se présente sous la forme d'un liquide homogène limpide et jaune contenant 0,5 % de MAM résiduel.

### Exemple 5

Dans un réacteur agité chauffé par double enveloppe, on charge 600 kg de NORSOCRYL^{©}104 et on porte la température dans le réacteur aux environs de 38°C (pression tête de colonne 50 mm Hg) tout en distillant une partie du MAM contenu dans le NORSOCRYL^{©}104. Lorsque la quantité de MAM distillée atteint 150 kg, on charge 150 kg d'AISOBOR dans le réacteur. On poursuit la distillation du MAM en abaissant progressivement la pression de service jusqu'à 30 mm Hg en tête de colonne afin de ne pas dépasser 50°C dans le réacteur. Lorsque la température du réacteur atteint 50°C, on introduit en une fois 100 kg d'AISOBOR. La quantité de MAM qui a été distillée durant cette étape est de 103 kg. On poursuit à nouveau la distillation du MAM en baissant progressivement la pression jusqu'à 20 mm Hg en tête de colonne. Lorsque la température du réacteur atteint 70°C, le MAM supplémentaire distillé représente 38,5 kg. On introduit alors 41,9 kg d'AISOBOR dans le réacteur. On effectue ensuite un strippage afin d'éliminer le MAM résiduel. Durant toute la durée de l'opération, la colonne est stabilisée avec une solution d'EMHQ dans l'AISOBOR et un bullage d'air appauvri à 7% d'oxygène en volume. est effectué dans le réacteur.

Le mélange final contient :
- MEIO : 40 %
- AISOBOR : 50,0 %
- MAM : 0,7 %
- Autres : 9,3 %
- EMHQ : 0,015 %
- PTZ : 0,035 %

## Revendications

1. Composition comprenant un (méth)acrylate d'alkylimidazolidone de formule (I) 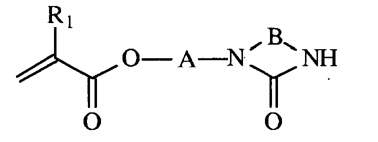 dans laquelle R₁ est un atome d'hydrogène ou un groupement méthyle, et A et B représentent, indépendamment l'un de l'autre, un groupement alkylène à chaîne droite ou ramifiée ayant de 2 à 5 atomes de carbone,
en solution dans un (méth)acrylate de formule (IV) 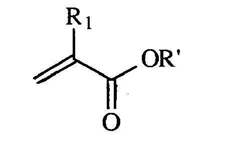 dans laquelle R₁ est un atome d'hydrogène ou un groupement méthyle, et R' est un radical alkyle à chaîne droite ou ramifiée ayant de 9 à 40 atomes de carbone ou un radical aliphatique cyclique, alcényle, aryle, alkyle-aryle ou aryle alkyle ayant de 2 à 40 atomes de carbone, ces radicaux pouvant être substitués et pouvant contenir des hétéroatomes,
et comprenant en outre un inhibiteur de polymérisation.

2. Composition selon la revendication 1 comprenant de 20 à 80 % en poids de (méth)acrylate d'alkylimidazolidone de formule (I) et de sous-produits monomériques porteurs d'une fonction uréido, de préférence de 30 à 60 % en poids.

3. Composition selon la revendication 1 ou 2, **caractérisée en ce que** le composé de formule (I) est le méthacrylate d'éthyl-1- imidazolidyl-2-one.

4. Composition selon l'une des revendications 1 à 3, **caractérisée en ce que** dans la formule (IV), le radical R' est lauryle, stéaryle, dodécyle, béhényle, isobornyle, phényle, naphtyle, naphtyloxyalkyle comprenant un groupement alkyle linéaire ou ramifié ayant de 1 à 10 atomes de carbone.

5. Composition selon l'une des revendications 1 à 4, **caractérisée en ce que** le (méth)acrylate (IV) est l'acrylate d'isobornyle.

6. Composition selon l'une des revendications 1 à 5, **caractérisé en ce que** l'inhibiteur de polymérisation est la phénothiazine, l'hydroquinone l'éther monométhylique d'hydroquinone, le diterbutyl para-crésol, la paraphénylène diamine, le TEMPO (2,2,6,6-tétraméthyl-1-piperidinyloxy) et le di-tertiobutylcatéchol, ou les dérivés du TEMPO

7. Composition selon l'une des revendications 1 à 6, **caractérisée en ce que** la teneur en inhibiteur de polymérisation est comprise entre 100 et 2000 ppm par rapport au mélange final, de préférence entre 200 et 600 ppm.

8. Procédé de préparation de solutions de (méth)acrylate d'alkylimidazolidone de formule (I) 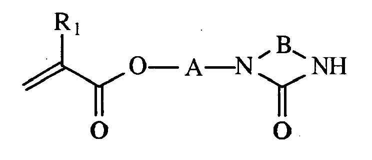 dans laquelle R₁ est un atome d'hydrogène ou un groupement méthyle, et A et B représentent, indépendamment l'un de l'autre, un groupement alkylène à chaîne droite ou ramifiée ayant de 2 à 5 atomes de carbone,
dans un (méth)acrylate de formule (IV) 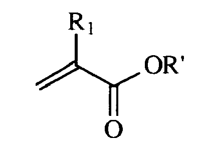 dans laquelle R₁ est un atome d'hydrogène ou un groupement méthyle, et R' est un radical alkyle à chaîne droite ou ramifiée ayant de 9 à 40 atomes de carbone ou un radical aliphatique cyclique, alcényle, aryle, alkyle-aryle ou aryle alkyle ayant de 2 à 40 atomes de carbone, ces radicaux pouvant être substitués et pouvant contenir des hétéroatomes,
à partir de solutions de (méth)acrylate d'alkylimidazolidone de formule (I) dans l'eau ou dans un (méth)acrylate de formule (II) 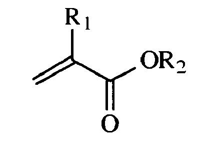 dans laquelle R₁ est un atome d'hydrogène ou un groupement méthyle et R₂ est un groupement alkyle à chaîne droite ou ramifiée ayant de 1 à 4 atomes de carbone.

9. Procédé selon la revendication 8, **caractérisé en ce que** le (méth)acrylate d'alkylimidazolidone est le méthacrylate d'éthyl-1- imidazolidyl-2-one.

10. Procédé selon la revendications 8 ou 9, **caractérisé en ce que** le (méth)acrylate (IV) est l'acrylate d'isobornyle.

11. Utilisation d'une composition selon l'une des revendications 1 à 7, ou d'une solution obtenue par le procédé selon l'une des revendications 8 à 10, dans la préparation de polymères utilisables comme revêtements et d'adhésifs, dans le traitement du papier et des textiles, comme agents de traitement du cuir et dans la production de peintures ou vernis polymérisables par chauffage ou sous radiation UV ou visible, ou sous faisceau d'électrons.

12. Composition comprenant de 45 à 55 % en poids de méthacrylate d'éthyl-1-imidazolidyl-2-one et de sous-produits monomériques porteurs d'une fonction uréido, et de 55 à 45 % en poids de (méth)acrylate d'isobornyle.

## Claims

1. A composition comprising an alkylimidazolidone (meth)acrylate of formula (I): 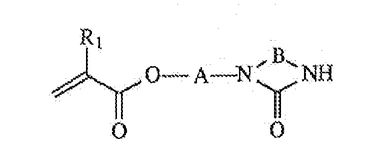 in which R₁ is a hydrogen atom or a methyl group and A and B represent, independently of one another, a straight- or branched-chain alkylene group having from 2 to 5 carbon atoms,
in solution in to (meth)acrylate of formula (IV) : 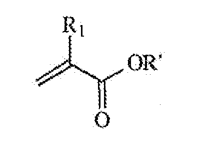 in which R₁ is a hydrogen atom or a methyl group and R' is a straight -- or branched-chain alkyl radical having from 9 to 40 carbon atoms or a cyclic aliphatic, alkenyl, aryl, alkylaryl or arylalkyl radical having from 2 to 40 carbon atoms, it being possible for these radicals to be substituted and to comprise heteroatoms,
and additionally comprising a polymerization inhibitor.

2. The composition as claimed in claim 1, comprising from 20 to 80% by weight of alkylimidazolidone (meth)acrylate of formula (I) and of monomeric byproducts carrying a ureido functional group, preferably from 30 to 60% by weight.

3. The composition as claimed in claim 1 or 2, **characterized in that** the compound of formula (I) is imidazolidyl-2-one-1-ethyl methacrylate.

4. The composition as claimed in one of claims 1 to 3, **characterized in that**, in the formula (IV), the radical R' is lauryl, stearyl, dodecyl, behenyl, isobornyl, phenyl, naphthyl or naphthyloxyalkyl comprising a linear or branched alkyl group having from 1 to 10 carbon atoms.

5. The composition as claimed in one of claims 1 to 4, **characterized in that** the (meth)acrylate (IV) is isobornyl acrylate.

6. The composition as claimed in one of claims 1 to 5, **characterized in that** the polymerization inhibitor is phenothiazine, hydroquinone, hydroquinone monomethyl ether, di (tert-butyl)-para-cresol, para-phenylenediamine, TEMPO (2, 2, 6, 6-tetramethyl-1-piperidinyloxy) and di(tert-butyl)catechol, or TEMPO derivatives.

7. The composition as claimed in one of claims 1 to 6, **characterized in that** the content of polymerization inhibitor is between 100 and 2000 ppm, with respect to the final mixture, preferable from 200 to 600 ppm.

8. A process for the preparation of solutions of alkylimidazolidone (meth)acrylate of formula (I): 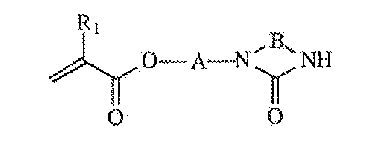 in which R₁ is a hydrogen atom or a methyl group and A and B represent, independently of one another, a straight- or branched-chain alkylene group having from 2 to 5 carbon atoms, in a (meth)acrylate of formula (IV): 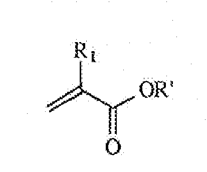 in which R₁ is a hydrogen atom or a methyl group and R' is a straight" or branched-chain alkyl radical having from 9 to 40 carbon atoms or a cyclic aliphatic, alkenyl, acryl, alkylaryl or arylalkyl radical having from 2 to 40 carbon atoms, it being possible for these radicals to be substituted and to comprise heteroatoms,
starting from solutions of alkylimidazolidone (meth) acrylate of formula (I) in water or in a (meth)acrylate of formula (II): 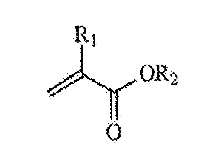 in which R₁ is a hydrogen atom or a methyl group and R₂ is a straight- or branched-chain alkyl group having from 1 to 4 carbon atoms.

9. The process as claimed in claim 8, **characterized in that** the alkylimidazolidone (meth) acrylate is imidazolidyl-2-one-1-ethyl methacrylate.

10. The process as claimed in claim 8 or 9, **characterised in that** the (meth) acrylate (IV) is isobornyl acrylate.

11. The use of a composition as claimed in one of claims 1 to 7 or of a solution obtained by the prowess as claimed in one of claims 8 to 10 in the preparation of polymers which can be used as coatings and adhesives, in the treatment of paper and textiles, as leather treatment agents and in the production of paints or varnishes which can be polymerized by heating or under UV or visible radiation or under a beam of electrons.

12. A composition comprising from 45 to 55% by weight of imidazolidyl-2-one-1-ethyl methacrylate and of monomeric by products carrying a ureido functional group and from 55 to 45% by weight of isobornyl (meth)acrylate.

## Patentansprüche

1. Zusammensetzung, die ein Alkylimidazolidon(meth)acrylat der Formel (I) enthält: 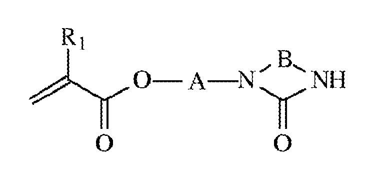 worin R₁ ein Wasserstoffatom oder die Methylgruppe bedeutet und A et B unabhängig voneinander eine Alkylengruppe mit gerader oder verzweigter Kette bedeuten, die 2 bis 5 Kohlenstoffatome aufweist, in Lösung in einem (Meth)acrylat der Formel (IV): 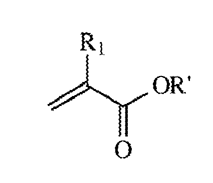 worin R₁ ein Wasserstoffatom oder die Methylgruppe bedeutet und R' eine Alkylgruppe mit gerader oder verzweigter Kette, die 9 bis 40 Kohlenstoffatome aufweist, oder eine cyclische aliphatische Gruppe, eine Alkenylgruppe, eine Arylgruppe, eine Alkylarylgruppe oder eine Arylalkylgruppe mit 2 bis 40 Kohlenstoffatomen ist, wobei diese Gruppen substituiert sein und Heteroatomen enthalten können, und
die ferner einen Polymerisationsinhibitor enthält.

2. Zusammensetzung nach Anspruch 1, die 20 bis 80 Gew.-% Alkylimidazolidon(meth)acrylat der Formel (I) und monomerer Nebenprodukte, die eine Ureidofunktion tragen, und vorzugsweise 30 bis 60 Gew.-% enthält.

3. Zusammensetzung nach Anspruch 1 oder 2, **dadurch gekennzeichnet, dass** die Verbindung der Formel (I) das 1-Ethylimidazolidyl-2-on-methacrylat ist.

4. Zusammensetzung nach einem der Ansprüche 1 bis 3, **dadurch gekennzeichnet, dass** in der Formel (IV) die Gruppe R' Lauryl, Stearyl, Dodecyl, Behenyl, Isobornyl, Phenyl, Naphtyl oder eine Naphtyloxyalkylgruppe bedeutet, die eine lineare oder verzweigte Alkylgruppe mit 1 bis 10 Kohlenstoffatomen umfasst.

5. Zusammensetzung nach einem der Ansprüche 1 bis 4, **dadurch gekennzeichnet, dass** das (Meth)acrylat (IV) das Isobornylacrylat ist.

6. Zusammensetzung nach einem der Ansprüche 1 bis 5, **dadurch gekennzeichnet, dass** es sich bei dem Polymerisationsinhibitor um Phenotiazin, Hydrochinon, Hydrochinonmonomethylether, Di-tert-butyl-kresol, p-Phenylendiamin, TEMPO (2,2,6,6-Tetramethyl-1-piperidinyloxid) und Di-tert-butylcatechol oder TEMPO-Derivate handelt.

7. Zusammensetzung nach einem der Ansprüche 1 bis 6, **dadurch gekennzeichnet, dass** der Mengenanteil des Polymerisationsinhibitors im Bereich von 100 bis 2000 ppm, bezogen auf das fertige Gemisch, und vorzugsweise im Bereich von 200 bis 600 ppm liegt.

8. Verfahren zur Herstellung von Lösungen des Alkylimidazolidon(meth)acrylats der Formel (I): 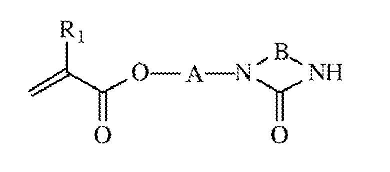 worin R₁ ein Wasserstoffatom oder die Methylgruppe bedeutet und A et B unabhängig voneinander eine Alkylengruppe mit gerader oder verzweigter Kette und mit 2 bis 5 Kohlenstoffatomen bedeuten,
in einem (Meth)acrylat der Formel (IV): 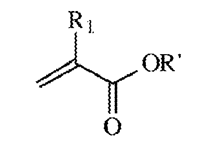 worin R₁ ein Wasserstoffatom oder die Methylgruppe bedeutet und R' eine Alkylgruppe mit gerader oder verzweigter Kette, die 9 bis 40 Kohlenstoffatome aufweist, oder eine cyclische aliphatische Gruppe, eine Alkenylgruppe, eine Arylgruppe, eine Alkylarylgruppe oder eine Arylalkylgruppe mit 2 bis 40 Kohlenstoffatomen ist, wobei diese Gruppen substituiert sein und Heteroatomen enthalten können,
ausgehend von Lösungen des Alkylimidazolidon(meth)acrylats der Formel (I) in Wasser oder in einem (Meth)acrylat der Formel (II): 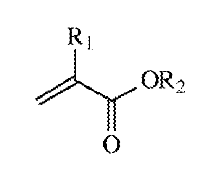 worin R₁ ein Wasserstoffatom oder die Methylgruppe bedeutet und R₂ eine Alkylgruppe mit gerader oder verzweigter Kette ist, die 1 bis 4 Kohlenstoffatome aufweist.

9. Verfahren nach Anspruch 8, **dadurch gekennzeichnet, dass** das Alkylimidazolidon(meth)acrylat das 1-Ethyl-imidazolidyl-2-on-methacrylat ist.

10. Verfahren nach Anspruch 8 oder 9, **dadurch gekennzeichnet, dass** (Meth)acrylat (IV) das Isobornylacrylat ist.

11. Verwendung einer Zusammensetzung nach einem der Ansprüche 1 bis 7 oder einer nach dem Verfahren nach einem der Ansprüche 8 bis 10 erhaltenen Lösung bei der Herstellung von Polymeren, die als Beschichtungen und Klebstoffe, bei der Behandlung von Papier und Textilien, als Agentien zur Behandlung von Leder und bei der Herstellung von Farben und Lacken verwendbar sind und durch Erwärmen oder durch Bestrahlen mit UV-Strahlung oder sichtbarem Licht oder Elektronenstrahlen polymerisierbar sind.

12. Zusammensetzung, die 45 bis 55 Gew.-% 1-Ethyl-imidazolidyl-2-on-methacrylat und monomerer Nebenprodukte mit Ureidofunktion und 55 bis 45 Gew.-% Isobornyl(meth)acrylat enthält.
